# EUROPEAN PATENT SPECIFICATION

(11) **EP 4 335 381 B1**
(45) Date of publication and mention of the grant of the patent: **24.12.2025**
(21) Application number: 23196399.2
(22) Date of filing: 08.09.2023
(51) Int. Cl.: A61B 8/08, A61B 8/00, G06T 7/00

(54) **ULTRASOUND DIAGNOSTIC APPARATUS AND CONTROL METHOD FOR ULTRASOUND DIAGNOSTIC APPARATUS**
ULTRASCHALLDIAGNOSEVORRICHTUNG UND STEUERUNGSVERFAHREN FÜR ULTRASCHALLDIAGNOSEVORRICHTUNG
APPAREIL DE DIAGNOSTIC À ULTRASONS ET PROCÉDÉ DE COMMANDE POUR APPAREIL DE DIAGNOSTIC À ULTRASONS

(30) Priority: 12.09.2022 JP 2022144569
(43) Date of publication of application: 13.03.2024
(73) Proprietor: FUJIFILM Corporation, Tokyo 106-8620 (JP)
(72) Inventor: MIYACHI, Yukiya, Kanagawa, 258-8538 (JP); EBATA, Tetsurou, Kanagawa, 258-8538 (JP)
(74) Representative: Dehns Germany Partnerschaft mbB

(56) References cited:
- US-A1- 2020 069 282
- US-A1- 2020 352 548

## Description

### BACKGROUND OF THE INVENTION

### 1. Field of the Invention

The present invention relates to an ultrasound diagnostic apparatus and a control method for an ultrasound diagnostic apparatus for setting a region of interest in an ultrasound image.

### 2. Description of the Related Art

Conventionally, an ultrasound image representing a tomogram of an inside of a subject under examination has been acquired using a so-called ultrasound diagnostic apparatus, and an examination for the subject under examination has been performed by a user such as a doctor based on the acquired ultrasound image. In order for the user to smoothly perform such an examination for the subject under examination, there is known a technique for automatically detecting or measuring an object such as an organ of the subject under examination shown in an ultrasound image. In this case, a so-called region of interest may be set in the ultrasound image in order to limit a range in which processing such as automatic detection or measurement is performed and to smoothly perform the processing. For example, WO2019/039028A discloses that a rectangular region of interest is set in an ultrasound image in order to limit a range in which a detection target is detected. US 2020/352548 A1, over which the independent claims are characterised, discloses an ultrasound imagining system wherein a part of a contour of a region of interest is displayed.

### SUMMARY OF THE DISCLOSURE

Meanwhile, in a case where a user such as a doctor confirms a detection target shown in an ultrasound image and performs a diagnosis, a region of interest including the detection target in the ultrasound image may be displayed on a monitor such that the detection target can be easily confirmed. Usually, the region of interest is often displayed with a closed rectangular frame line.

In addition, in a case where the detection target is a feces, a cyst, or the like present inside the rectum, it is known that in order to perform an accurate diagnosis, it is necessary to confirm not only the presence of the detection target but also whether or not there is a so-called posterior acoustic shadow in a region located at a deeper part than the detection target, whether or not a so-called posterior echo is enhanced, or the like. Therefore, in a case where the region of interest is displayed with the closed rectangular frame line, it may be difficult for the user to confirm the region located at the deeper part than the detection target in the ultrasound image, which may make it difficult to easily perform an accurate diagnosis.

The present invention has been made in order to solve such a conventional problem, and an object of the present invention is to provide an ultrasound diagnostic apparatus and a control method for an ultrasound diagnostic apparatus that enable a user to easily and accurately perform a diagnosis.

According to the following configuration, the above object can be achieved.

Therefore, the user can easily and accurately perform a diagnosis.

The invention is set out in the appended set of claims.

### BRIEF DESCRIPTION OF THE DRAWINGS

Fig. 1 is a block diagram showing a configuration of an ultrasound diagnostic apparatus according to an embodiment of the present invention.
Fig. 2 is a block diagram showing a configuration of a transmission/reception circuit in the embodiment of the present invention.
Fig. 3 is a block diagram showing a configuration of an image generation unit in the embodiment of the present invention.
Fig. 4 is a diagram showing a contour line of a detection target.
Fig. 5 is a diagram showing an image region including the detection target.
Fig. 6 is a diagram showing an example of a region of interest set for a feces shown in an ultrasound image.
Fig. 7 is a diagram showing a first example of a region-of-interest suggestion line in the embodiment of the present invention.
Fig. 8 is a diagram showing an example of the region-of-interest suggestion line set for a B-line in the embodiment of the present invention.
Fig. 9 is a flowchart showing an operation of the ultrasound diagnostic apparatus according to the embodiment of the present invention.
Fig. 10 is a diagram showing a second example of the region-of-interest suggestion line in the embodiment of the present invention.
Fig. 11 is a diagram showing a third example of the region-of-interest suggestion line in the embodiment of the present invention.
Fig. 12 is a diagram showing a fourth example of the region-of-interest suggestion line in the embodiment of the present invention.

### DESCRIPTION OF THE PREFERRED EMBODIMENTS

Hereinafter, embodiments of the present invention will be described with reference to the accompanying drawings.

In the present specification, a numerical range represented by "to" means a range including numerical values described before and after "to" as a lower limit value and an upper limit value, respectively.

In the present specification, "same" and "identical" include an error range generally allowed in the technical field.

### Embodiment

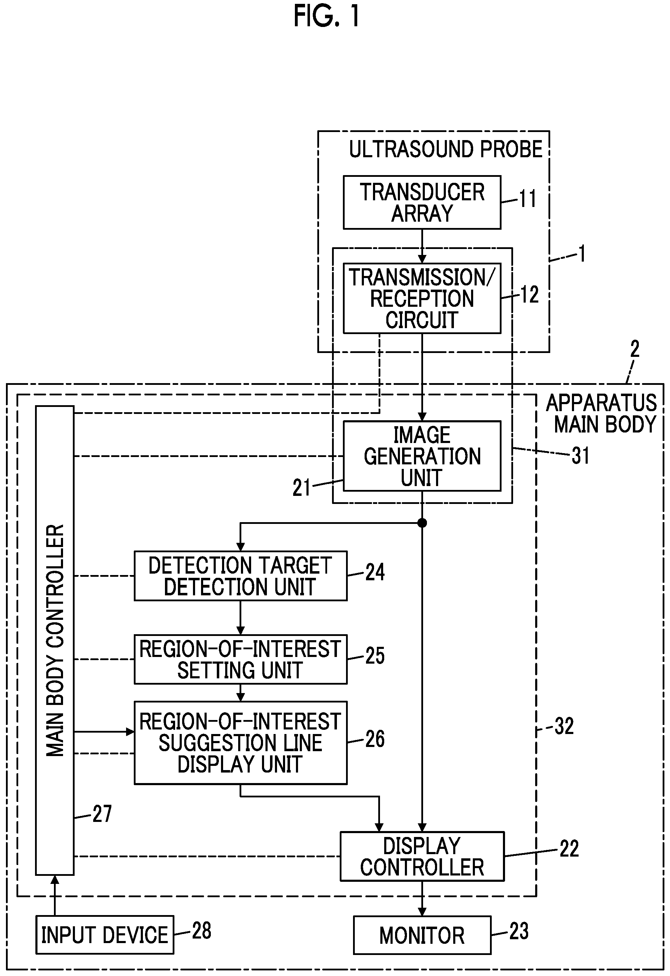

Fig. 1 shows a configuration of an ultrasound diagnostic apparatus according to the embodiment of the present invention. The ultrasound diagnostic apparatus comprises an ultrasound probe 1 and an apparatus main body 2 connected to the ultrasound probe 1.

The ultrasound probe 1 includes a transducer array 11. A transmission/reception circuit 12 is connected to the transducer array 11.

The apparatus main body 2 includes an image generation unit 21 connected to the transmission/reception circuit 12 of the ultrasound probe 1. A display controller 22 and a monitor 23 are sequentially connected to the image generation unit 21. In addition, a detection target detection unit 24, a region-of-interest setting unit 25, and a region-of-interest suggestion line display unit 26 are sequentially connected to the image generation unit 21. The region-of-interest suggestion line display unit 26 is connected to the display controller 22. In addition, a main body controller 27 is connected to the transmission/reception circuit 12, the image generation unit 21, the display controller 22, the detection target detection unit 24, the region-of-interest setting unit 25, and the region-of-interest suggestion line display unit 26. An input device 28 is connected to the main body controller 27.

In addition, the transmission/reception circuit 12 and the image generation unit 21 constitute an image acquisition unit 31. Further, the image generation unit 21, the display controller 22, the detection target detection unit 24, the region-of-interest setting unit 25, the region-of-interest suggestion line display unit 26, and the main body controller 27 constitute a processor 32 for the apparatus main body 2.

The transducer array 11 of the ultrasound probe 1 includes a plurality of ultrasound transducers one-dimensionally or two-dimensionally arranged. Each of these ultrasound transducers transmits an ultrasound wave in accordance with a drive signal supplied from the transmission/reception circuit 12 and receives an ultrasound echo from a subject under examination to output a signal based on the ultrasound echo. For example, each ultrasound transducer is composed of a piezoelectric material consisting of piezoelectric ceramic represented by lead zirconate titanate (PZT), a polymer piezoelectric element represented by poly vinylidene di fluoride (PVDF), piezoelectric single crystal represented by lead magnesium niobate-lead titanate (PMN-PT), or the like, and electrodes formed at both ends of the piezoelectric material.

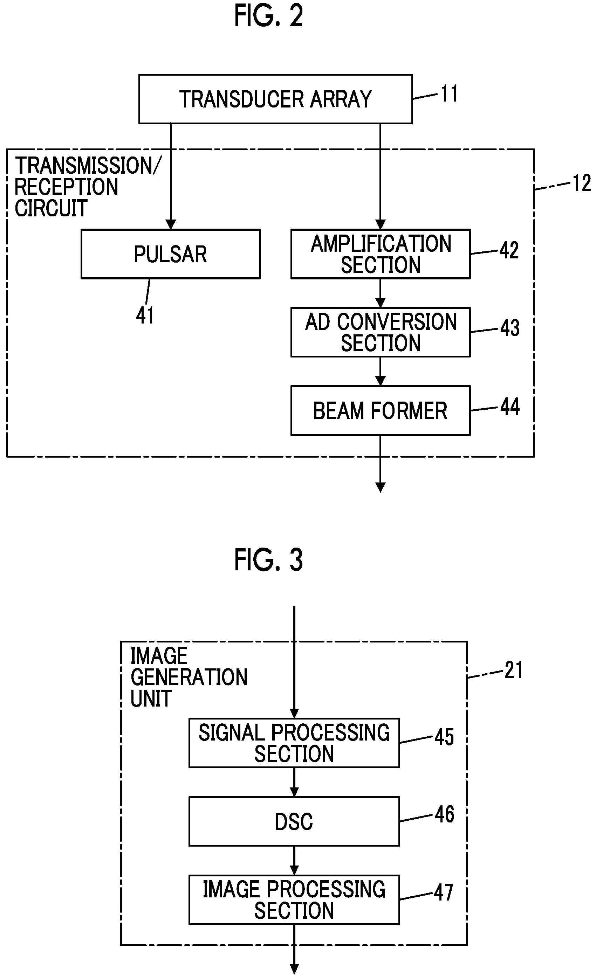

Under the control of the main body controller 27, the transmission/reception circuit 12 transmits the ultrasound wave from the transducer array 11 and generates a sound ray signal based on a reception signal acquired by the transducer array 11. As shown in Fig. 2, the transmission/reception circuit 12 includes a pulsar 41 connected to the transducer array 11, an amplification section 42, an analog-to-digital (AD) conversion section 43, and a beam former 44 that are sequentially connected in series to the transducer array 11.

The pulsar 41 includes, for example, a plurality of pulse generators, and the pulsar 41 adjusts an amount of delay of each of drive signals and supplies the drive signals to the plurality of ultrasound transducers such that ultrasound waves transmitted from the plurality of ultrasound transducers of the transducer array 11 form an ultrasound beam based on a transmission delay pattern selected according to a control signal from the main body controller 27. In this way, in a case where a pulsed or continuous-wave voltage is applied to the electrodes of the ultrasound transducer of the transducer array 11, the piezoelectric material expands and contracts to generate pulsed or continuous-wave ultrasound wave from each of the ultrasound transducers, whereby an ultrasound beam is formed from the combined wave of these ultrasound waves.

The transmitted ultrasound beam is reflected in, for example, a target such as a site of the subject under examination and propagates toward the transducer array 11 of the ultrasound probe 1. The ultrasound echo propagating toward the transducer array 11 in this way is received by each of the ultrasound transducers constituting the transducer array 11. In this case, each of the ultrasound transducers constituting the transducer array 11 receives the propagating ultrasound echo to expand and contract to generate a reception signal, which is an electrical signal, and outputs these reception signals to the amplification section 42.

The amplification section 42 amplifies the signal input from each of the ultrasound transducers constituting the transducer array 11 and transmits the amplified signal to the AD conversion section 43. The AD conversion section 43 converts the signal transmitted from the amplification section 42 into digital reception data. The beam former 44 performs so-called reception focus processing by applying and adding a delay to each reception data received from the AD conversion section 43. By this reception focus processing, each reception data converted by the AD conversion section 43 is phase-added, and a sound ray signal in which the focus of the ultrasound echo is narrowed down is acquired.

As shown in Fig. 3, the image generation unit 21 has a configuration in which a signal processing section 45, a digital scan converter (DSC) 46, and an image processing section 47 are sequentially connected in series.

The signal processing section 45 generates a B-mode image signal, which is tomographic image information regarding tissues inside the subject under examination, by performing, on the sound ray signal received from the transmission/reception circuit 12, correction of the attenuation due to the distance according to the depth of the reflection position of the ultrasound wave using a sound speed value set by the main body controller 27 and then performing envelope detection processing.

The DSC 46 converts (raster-converts) the B-mode image signal generated by the signal processing section 45 into an image signal according to a normal television signal scanning method.

The image processing section 47 performs various types of necessary image processing such as gradation processing on the B-mode image signal input from the DSC 46 and then sends the B-mode image signal to the display controller 22 and the detection target detection unit 24. Hereinafter, the B-mode image signal that has been subjected to image processing by the image processing section 47 is referred to as an ultrasound image.

Under the control of the main body controller 27, the display controller 22 performs predetermined processing on the ultrasound image or the like generated by the image generation unit 21 and displays the ultrasound image or the like on the monitor 23.

The monitor 23 performs various kinds of display under the control of the display controller 22. The monitor 23 can include a display device such as a liquid crystal display (LCD), or an organic electroluminescence (EL) display, for example.

The detection target detection unit 24 analyzes the ultrasound image generated by the image generation unit 21 to detect the detection target captured in the ultrasound image. The detection target detection unit 24 stores, for example, a plurality of template images for each of a plurality of detection targets and can detect the detection target by searching within the ultrasound image according to a so-called template matching method using the plurality of template images. In addition, the detection target detection unit 24 includes, for example, a machine learning model that has learned a large number of ultrasound images showing the plurality of detection targets, respectively, and can also use this machine learning model to detect the detection target shown in the ultrasound image.

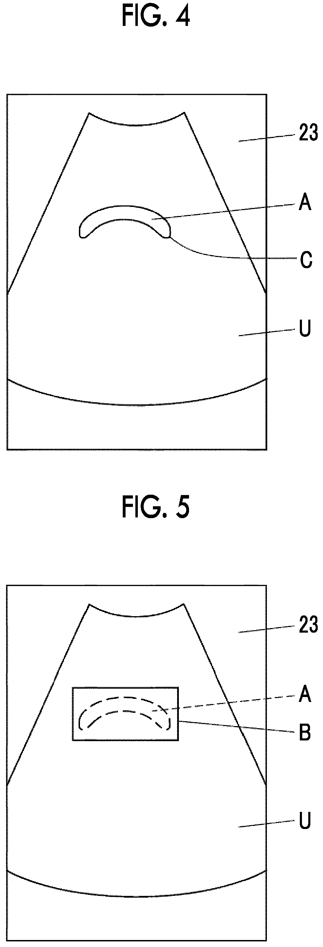

For example, as shown in Fig. 4, the detection target detection unit 24 can output a contour line C of a detection target A in an ultrasound image U as a detection result.

Instead of outputting the contour line C of the detection target A as the detection result, the detection target detection unit 24 can also output a rectangular image region B including the detection target A as the detection result, as shown in Fig. 5, for example. In this case, in order to accurately specify a position of the detection target A in a depth direction, it is preferable that a length of the image region B in the depth direction is at least 1 time and less than 2 times a length of the detection target A in the depth direction.

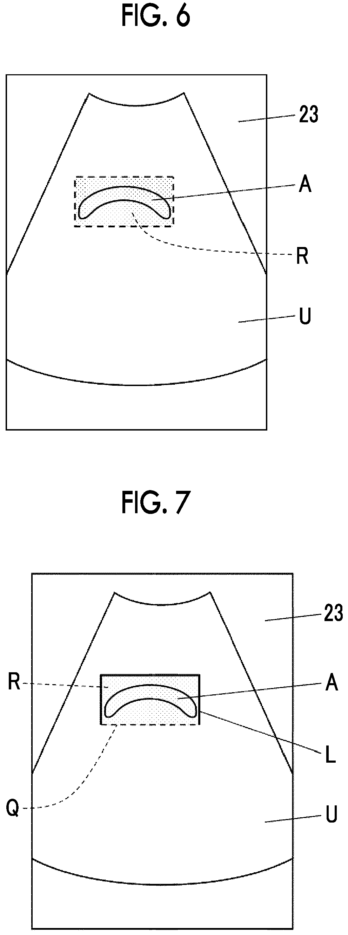

For example, as shown in Fig. 6, the region-of-interest setting unit 25 sets, on the ultrasound image U, a region of interest R including the detection target A detected by the detection target detection unit 24.

In a case where the detection target detection unit 24 outputs the contour line C of the detection target A, the region-of-interest setting unit 25 sets, for example, a rectangular region that includes the contour line C and that circumscribes the contour line C, and enlarges the rectangular region by a magnification of at least 1 time and less than 2 times, for example, by a magnification of 1.2 times, while fixing the position of the center of gravity of the rectangular region, whereby the region of interest R can be set.

In addition, in a case where the detection target detection unit 24 outputs the image region B including the detection target A instead of outputting the contour line C of the detection target A, the region-of-interest setting unit 25 can set the image region B as the region of interest R.

For example, as shown in Fig. 7, the region-of-interest suggestion line display unit 26 displays, on the monitor 23, a region-of-interest suggestion line L consisting of only a part of a contour line Q of the region of interest R set by the region-of-interest setting unit 25. Here, the contour line Q of the region of interest R refers to a closed frame line extending along the contour of the region of interest R. In the example of Fig. 7, the contour line Q of the region of interest R is a rectangular frame line extending along the contour of the rectangular region of interest R.

For example, as shown in Fig. 7, the region-of-interest suggestion line display unit 26 can display, as the region-of-interest suggestion line L, a line excluding a portion of the contour line Q of the region of interest R, which is a portion located at a deeper part than the detection target A.

Meanwhile, depending on the type of the detection target A, it may be necessary to confirm a so-called posterior echo in order to perform the diagnosis related to the detection target A. It is known that, for example, in a case of performing a diagnosis related to a feces present inside the rectum of the subject under examination, it is necessary to confirm the posterior echo of the feces to determine whether or not there is a so-called posterior acoustic shadow in a region located at the deeper part than the feces. The posterior acoustic shadow refers to a low brightness region located at the deeper part than the detection target A such as a feces in the ultrasound image U, which is caused by the ultrasound wave propagating from a body surface side of the subject under examination being unable to pass through the detection target A. The posterior acoustic shadow is often generated in the ultrasound image U in which the hard feces is captured, and is often confirmed in order to determine the property of the feces, that is, the hardness of the feces.

In the embodiment of the present invention, since the region-of-interest suggestion line L to be displayed on the monitor 23 by the region-of-interest suggestion line display unit 26 is composed of, for example, a line consisting of only a part of the contour line Q and excluding a portion of the contour line Q of the region of interest R, which is a portion located at the deeper part than the detection target A, the user such as a doctor can easily and accurately perform the diagnosis related to the detection target A by clearly confirming the posterior echo of the detection target A such as the posterior acoustic shadow.

In addition to the feces, examples of the detection target A for which the posterior echo needs to be confirmed include a cyst. In general, it is known that the posterior echo may be enhanced in the ultrasound image U in which the cyst is captured. The enhancement of the posterior echo refers to a high brightness region located at the deeper part than the detection target A in the ultrasound image U, where the attenuation of the ultrasound wave is small, such as a cyst. In this way, even in a case where the posterior echo of the detection target A is enhanced, the user can easily and accurately perform the diagnosis related to the detection target A by clearly confirming the enhancement of the posterior echo because the region-of-interest suggestion line L is composed of a line consisting of only a part of the contour line Q of the region of interest R.

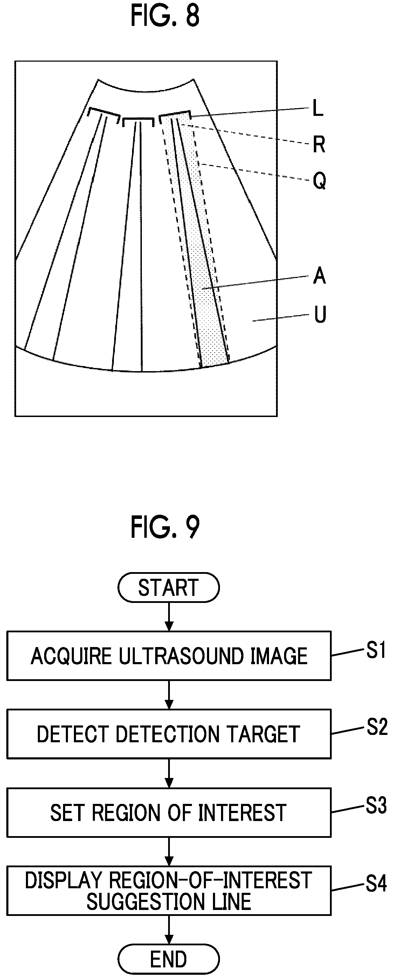

Further, in the ultrasound image U in which a so-called B-line in the lung echo is captured, for example, displaying the region of interest R of the B-line with a closed frame line surrounding the generation portion of the B-line may make it difficult for the user to confirm the continuity of the B-line. In addition, displaying the region of interest R of the B-line with a closed frame line surrounding the entire B-line may make the display range of the frame line too wide, which makes it difficult for the user to confirm the ultrasound image U. As shown in Fig. 8, even in a case where the detection target A is the B-line, the region-of-interest suggestion line display unit 26 constructs the region-of-interest suggestion line L with the line consisting of only a part of the contour line Q of the region of interest R, so that the user can easily and accurately perform a diagnosis related to the lung echo by clearly confirming the B-line.

The main body controller 27 controls each unit of the apparatus main body 2 and the ultrasound probe 1 in accordance with a program recorded in advance, or the like.

The input device 28 accepts an input operation by an examiner and sends input information to the main body controller 27. The input device 28 is composed of, for example, a device for the examiner to perform an input operation, such as a keyboard, a mouse, a trackball, a touchpad, or a touch panel.

Although the processor 32 including the image generation unit 21, the display controller 22, the detection target detection unit 24, the region-of-interest setting unit 25, the region-of-interest suggestion line display unit 26, and the main body controller 27 may be composed of a central processing unit (CPU) and a control program for causing the CPU to perform various types of processing, the processor 32 may be composed of a field programmable gate array (FPGA), a digital signal processor (DSP), an application specific integrated circuit (ASIC), a graphics processing unit (GPU), or other integrated circuits (ICs), or may be composed of a combination thereof.

In addition, the image generation unit 21, the display controller 22, the detection target detection unit 24, the region-of-interest setting unit 25, the region-of-interest suggestion line display unit 26, and the main body controller 27 of the processor 32 can also be configured by being integrated partially or entirely into one CPU or the like.

Next, an example of an operation of the ultrasound diagnostic apparatus according to the embodiment will be described with reference to the flowchart of Fig. 9.

First, in step S1, the ultrasound image U is acquired by the image acquisition unit 31. In this case, the transducer array 11 of the ultrasound probe 1 transmits the ultrasound beam into the subject under examination and receives the ultrasound echo from the inside of the subject under examination, whereby the reception signal is generated. The transmission/reception circuit 12 of the image acquisition unit 31 performs so-called reception focus processing on the reception signal to generate the sound ray signal, under the control of the main body controller 27. The sound ray signal generated by the transmission/reception circuit 12 is sent to the image generation unit 21. The image generation unit 21 generates the ultrasound image U using the sound ray signal sent from the transmission/reception circuit 12.

Next, in step S2, the detection target detection unit 24 analyzes the ultrasound image U acquired in step S1 to detect the detection target A shown in the ultrasound image U. In this case, the detection target detection unit 24 can detect the detection target A using a template matching method and can detect the detection target using a machine learning model that has learned in advance a large number of ultrasound images U showing the detection targets.

In step S3, the region-of-interest setting unit 25 sets, on the ultrasound image U, the region of interest R including the detection target A detected in step S2, for example, as shown in Fig. 6. In this case, the region-of-interest setting unit 25 can set the length of the region of interest R in the depth direction to be at least 1 time and less than 2 times the length of the detection target A in the depth direction such that the user can clearly grasp the position of the detection target A in the depth direction.

Finally, in step S4, the region-of-interest suggestion line display unit 26 displays the region-of-interest suggestion line L consisting of only a part of the contour line Q of the region of interest R set in step S3 on the monitor 23, as shown in Fig. 7, for example. As a result, even in a case where images extending toward the deeper part of the ultrasound image U, which are images useful in the diagnosis of the subject under examination, such as the posterior acoustic shadow, the enhancement of the posterior echo, and the B-line, are generated, the user such as a doctor can easily and accurately diagnose the subject under examination by clearly confirming these images without being obstructed by the region-of-interest suggestion line L.

In a case where the processing of step S4 is completed in this manner, the operation of the ultrasound diagnostic apparatus according to the flowchart of Fig. 9 is completed.

As described above, with the ultrasound diagnostic apparatus according to the embodiment of the present invention, the detection target detection unit 24 detects the detection target A captured in the ultrasound image U, the region-of-interest setting unit 25 sets, on the ultrasound image U, the region of interest R including the detection target A, and the region-of-interest suggestion line display unit 26 displays the region-of-interest suggestion line L consisting of only a part of the contour line Q of the region of interest R on the monitor 23. Therefore, even in a case where images that are useful in the diagnosis of the subject under examination, such as the posterior acoustic shadow, the enhancement of the posterior echo, and the B-line, are generated, the user can easily and accurately diagnose the subject under examination by clearly confirming these images.

Although a case where the transmission/reception circuit 12 is provided in the ultrasound probe 1 has been described, the transmission/reception circuit 12 may be provided in the apparatus main body 2.

Further, although a case where the image generation unit 21 is provided in the apparatus main body 2 has been described, the image generation unit 21 may be provided in the ultrasound probe 1.

In addition, the apparatus main body 2 may be a so-called stationary type, a portable type that is easy to carry, or a so-called handheld type that is composed of, for example, a smartphone or a tablet type computer. As described above, the type of the device that constitutes the apparatus main body 2 is not particularly limited.

In addition, the apparatus main body 2 can also comprise an image memory (not shown) that stores the ultrasound image U generated by the image generation unit 21 for each examination. In this case, the processing of steps S2 to S4 in the flowchart of Fig. 9 can also be performed, for example, on the ultrasound image U acquired in the past examination and stored in the image memory based on the user's instruction via the input device 28.

In addition, the apparatus main body 2 can also comprise an image input unit (not shown) for inputting the ultrasound image U from an external device (not shown). In this case, the processing of steps S2 to S4 in the flowchart of Fig. 9 can also be performed, for example, on the ultrasound image U input from the external device via the image input unit based on the user's instruction via the input device 28.

Further, a case where the region-of-interest suggestion line display unit 26 can display, as the region-of-interest suggestion line L, a line excluding a portion of the contour line Q of the region of interest R, which is a portion located at the deeper part than the detection target A, on the monitor 23 has been described, but the aspect of the region-of-interest suggestion line L is not particularly limited thereto.

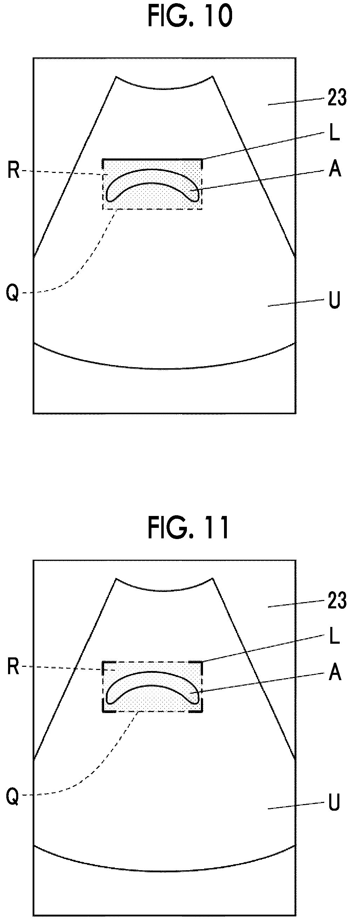

For example, as shown in Fig. 10, the region-of-interest suggestion line L can also consist of a portion of the contour line Q of the region of interest R, which is a portion located at a shallower part than the detection target A.

In addition, in a case where the region of interest R has the shape of a quadrangle, for example, as shown in Fig. 11, the region-of-interest suggestion line L can also consist of four portions disposed at positions of four vertices of the quadrangle. Fig. 11 shows that the region of interest R is a rectangle, but even in a case where the shape of the region of interest R is, for example, a quadrangle other than the rectangle, such as a trapezoid, the region-of-interest suggestion line L can also consist of four portions disposed at positions of four vertices of the quadrangle.

Here, the quadrangle may be a quadrangle having corners with right angles at the positions of the four vertices, or a so-called rounded quadrangle having rounded corners with a certain curvature. For example, in a case where a plurality of detection targets A such as an empty rectum and a feces are set, a quadrangle having corners with right angles can be set as the region of interest R for the empty rectum, and a rounded quadrangle can be set as the region of interest R for the feces. In this case, for example, the region-of-interest suggestion line L consisting of four corners with right angles can be set for the empty rectum, and the region-of-interest suggestion line L consisting of four rounded corners can be set for the feces. As a result, the user can grasp the type of the detection target A for which the region of interest R is set by confirming the shape of the region-of-interest suggestion line L.

Further, a case where the region of interest R to be set by the region-of-interest setting unit 25 has a rectangular shape has been described, but the shape of the region of interest R is not limited to the rectangular shape and can be any shape, such as a circular shape and a polygonal shape.

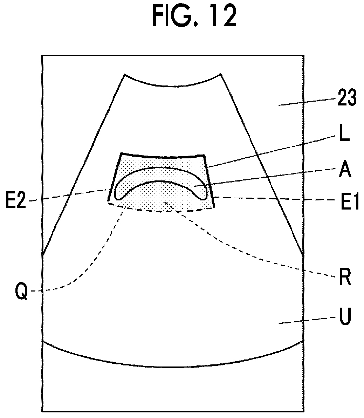

For example, as shown in Fig. 12, the region of interest R can have two sides E1 and E2 that extend along the sound line of the ultrasound image U and that face each other. Since images such as the posterior acoustic shadow, the enhanced posterior echo, and the B-line are usually formed along the sound line, the region of interest R has two sides E1 and E2 extending along the sound line, so that the region-of-interest suggestion line L does not overlap with images such as the posterior acoustic shadow, the enhanced posterior echo, and the B-line, and the user can confirm these images more clearly.

In addition, the shape and size of the region of interest R can also be decided on, for example, based on an input operation of the user via the input device 28. In this case, the region-of-interest setting unit 25 can set, on the ultrasound image U, the region of interest R having at least one of the shape or the size decided on based on the input operation of the user via the input device 28.

Further, a case where the detection target detection unit 24 detects the detection target A by analyzing the ultrasound image U generated by the image generation unit 21 has been described, but the detection target detection unit 24 also can detect the detection target A by, for example, analyzing the B-mode image signal generated by the signal processing section 45. In this case, the region-of-interest setting unit 25 can set the region of interest R for the B-mode image signal generated by the signal processing section 45.

In a case where the B-mode image signal for which the region of interest R is set in this manner undergoes processing by the DSC 46 and the image processing section 47, the region of interest R set for the B-mode image signal is converted into a shape along the sound line as shown in Fig. 12. Even in a case where the region of interest R is set in this manner, the region-of-interest suggestion line L consisting of only a part of the contour line Q of the region of interest R is displayed on the monitor 23 by the region-of-interest suggestion line display unit 26. Therefore, even in a case where images that are useful in the diagnosis of the subject under examination, such as the posterior acoustic shadow, the enhancement of the posterior echo, and the B-line, are generated, the user can easily and accurately diagnose the subject under examination by clearly confirming these images.

### Explanation of References

1: ultrasound probe
2: apparatus main body
11: transducer array
12: transmission/reception circuit
21: image generation unit
22: display controller
23: monitor
24: detection target detection unit
25: region-of-interest setting unit
26: region-of-interest suggestion line display unit
27: main body controller
28: input device
31: image acquisition unit
32: processor
41: pulsar
42: amplification section
43: AD conversion section
44: beam former
45: signal processing section
46: DSC
47: image processing section
A: detection target
B: image region
C, Q: contour line
E1, E2: side
L: region-of-interest suggestion line
R: region of interest
U: ultrasound image

## Claims

1. An ultrasound diagnostic apparatus comprising:
a monitor (23);
a detection target detection unit (24) configured to detect a B-line in a lung echo as a detection target (A) captured in an ultrasound image;
a region-of-interest setting unit (25) configured to set, on the ultrasound image, a region of interest (R) including the B-line detection target detected by the detection target detection unit (24); and
**characterized in that** the ultrasound diagnostic apparatus further comprises a region-of-interest suggestion line display unit (26) configured to display, on the monitor, a region-of-interest suggestion line (L) consisting of only a part of a contour line of the region of interest set by the region-of-interest setting unit (25), the part located at a shallower part than the B-line.

2. The ultrasound diagnostic apparatus according to claim 1,
wherein the region-of-interest suggestion line (L) is a line excluding a portion of the contour line of the region of interest, the portion being located at a deeper part than the detection target.

3. The ultrasound diagnostic apparatus according to claim 1,
wherein the region of interest has a shape of a quadrangle, and
the region-of-interest suggestion line consists of four portions disposed at positions of four vertices of the quadrangle.

4. The ultrasound diagnostic apparatus according to claim 3,
wherein the quadrangle has rounded corners at the positions of the four vertices.

5. The ultrasound diagnostic apparatus according to any one of claims 1 to 4,
wherein the region of interest has two sides that extend along a sound line in the ultrasound image and that face each other.

6. The ultrasound diagnostic apparatus according to any one of claims 1 to 7, further comprising:
an input device (28) configured to accept an input operation of a user,
wherein the region-of-interest setting unit (25) is configured to set the region of interest having at least one of a shape or a size decided based on the input operation of the user via the input device.

7. The ultrasound diagnostic apparatus according to any one of claims 1 to 8, further comprising:
an ultrasound probe (1); and
an image acquisition unit (31) configured to acquire the ultrasound image using the ultrasound probe (1).

8. A control method for an ultrasound diagnostic apparatus, comprising:
detecting a B-line in a lung echo as detection target captured in an ultrasound image;
setting, on the ultrasound image, a region of interest including the B-line detected detection target; and
**characterized in that** the control method further comprises displaying, on a monitor, a region-of-interest suggestion line consisting of only a part of a contour line of the region of interest, the part being located at a shallower part than the B-line.

## Patentansprüche

1. Ultraschalldiagnosevorrichtung, umfassend:
einen Monitor (23);
eine Detektionsziel-Detektionseinheit (24), die so konfiguriert ist, dass sie eine B-Linie in einem Lungenultraschall als ein Detektionsziel (A), das in einem Ultraschallbild aufgenommen ist, detektiert;
eine-Einstelleinheit (25) für Bereich von Interesse, die so konfiguriert ist, dass sie auf dem Ultraschallbild einen Bereich von Interesse (R), der das von der Detektionsziel-Detektionseinheit (24) detektierte B-Linien-Detektionsziel enthält, einstellt; und
**dadurch gekennzeichnet, dass** die Ultraschalldiagnosevorrichtung ferner umfasst:
eine Vorschlagslinie-Anzeigeeinheit (26) für Bereich von Interesse, die so konfiguriert ist, dass sie auf dem Monitor eine-Vorschlagslinie (L) für Bereich von Interesse, die nur aus einem Teil einer Konturlinie des von der Einstelleinheit (25) für Bereich von Interesse eingestellten Bereichs von Interesse besteht, anzeigt, wobei sich der Teil an einem flacheren Teil als die B-Linie befindet.

2. Ultraschalldiagnosevorrichtung nach Anspruch 1,
wobei die Vorschlagslinie (L) für Bereich von Interesse eine Linie ist, die einen Abschnitt der Konturlinie des Bereichs von Interesse ausschließt, wobei sich der Abschnitt an einem tieferen Teil als das Detektionsziel befindet.

3. Ultraschalldiagnosevorrichtung nach Anspruch 1,
wobei der Bereich von Interesse eine Form eines Vierecks aufweist, und
die Vorschlagslinie für Bereich von Interesse aus vier Abschnitten, die an Positionen von vier Ecken des Vierecks angeordnet sind, besteht.

4. Ultraschalldiagnosevorrichtung nach Anspruch 3,
wobei das Viereck an den Positionen der vier Ecken abgerundete Ecken aufweist.

5. Ultraschalldiagnosevorrichtung nach einem der Ansprüche 1 bis 4,
wobei der Bereich von Interesse zwei Seiten, die sich entlang einer Schalllinie in dem Ultraschallbild erstrecken und die einander zugewandt sind, aufweist.

6. Ultraschalldiagnosevorrichtung nach einem der Ansprüche 1 bis 7, ferner umfassend:
eine Eingabevorrichtung (28), die so konfiguriert ist, dass sie eine Eingabebedienung eines Benutzers akzeptiert,
wobei die Einstelleinheit (25) für Bereich von Interesse so konfiguriert ist, dass sie den Bereich von Interesse einstellt, der mindestens eine von einer Form und einer Größe aufweist, die auf der Grundlage der Eingabebedienung des Benutzers via die Eingabevorrichtung entschieden wird.

7. Ultraschalldiagnosevorrichtung nach einem der Ansprüche 1 bis 8, ferner umfassend:
eine Ultraschallsonde (1); und
eine Bilderfassungseinheit (31), die so konfiguriert ist, dass sie das Ultraschallbild unter Verwendung der Ultraschallsonde (1) erfasst.

8. Steuerverfahren für eine Ultraschalldiagnosevorrichtung, umfassend:
Detektieren einer B-Linie in einem Lungenultraschall als Detektionsziel, das in einem Ultraschallbild aufgenommen wird;
Einstellen, auf dem Ultraschallbild, eines Bereichs von Interesse, der das detektierte Detektionsziel enthält; und
**dadurch gekennzeichnet, dass** das Steuerverfahren ferner umfasst:
Anzeigen einer Vorschlagslinie für Bereich von Interesse, die nur aus einem Teil einer Konturlinie des Bereichs von Interesse besteht, wobei sich der Teil an einem flacheren Teil als der B-Linie befindet, auf einem Monitor.

## Revendications

1. Appareil de diagnostic par ultrasons comprenant :
un moniteur (23);
une unité de détection de cible de détection (24) configurée pour détecter une ligne B dans un écho pulmonaire en tant que cible de détection (A) capturée dans une image ultrasonore ;
une unité de définition de région d'intérêt (25) configurée pour définir, sur l'image ultrasonore, une région d'intérêt (R) incluant la cible de détection de ligne B détectée par l'unité de détection de cible de détection (24) ; et
**caractérisé en ce que** l'appareil de diagnostic par ultrasons comprend en outre
une unité d'affichage de ligne de suggestion de région d'intérêt (26) configurée pour afficher, sur le moniteur, une ligne de suggestion de région d'intérêt (L) constituée uniquement d'une partie d'une ligne de contour de la région d'intérêt définie par l'unité de définition de région d'intérêt (25), la partie étant située dans une partie plus superficielle que la ligne B.

2. Appareil de diagnostic par ultrasons selon la revendication 1,
dans lequel la ligne de suggestion de région d'intérêt (L) est une ligne excluant une portion de la ligne de contour de la région d'intérêt, la portion étant située dans une partie plus profonde que la cible de détection.

3. Appareil de diagnostic par ultrasons selon la revendication 1,
dans lequel la région d'intérêt a une forme d'un quadrilatère, et
la ligne de suggestion de région d'intérêt est constituée de quatre portions disposées à des positions de quatre sommets du quadrilatère.

4. Appareil de diagnostic par ultrasons selon la revendication 3,
dans lequel le quadrilatère a des coins arrondis aux positions des quatre sommets.

5. Appareil de diagnostic par ultrasons selon l'une quelconque des revendications 1 à 4, dans lequel la région d'intérêt comporte deux côtés qui s'étendent le long d'une ligne de son dans l'image ultrasonore et qui se font face.

6. Appareil de diagnostic par ultrasons selon l'une quelconque des revendications 1 à 7, comprenant en outre :
un dispositif d'entrée (28) configuré pour accepter une opération d'entrée d'un utilisateur,
dans lequel l'unité de définition de région d'intérêt (25) est configurée pour définir la région d'intérêt ayant au moins l'une d'une forme ou d'une taille déterminée sur la base de l'opération d'entrée de l'utilisateur via le dispositif d'entrée.

7. Appareil de diagnostic par ultrasons selon l'une quelconque des revendications 1 à 8, comprenant en outre :
une sonde ultrasonore (1) ; et
une unité d'acquisition d'images (31) configurée pour acquérir l'image ultrasonore à l'aide de la sonde ultrasonore (1).

8. Procédé de contrôle pour un appareil de diagnostic par ultrasons, comprenant :
détecter une ligne B dans un écho pulmonaire en tant que cible de détection capturée dans une image ultrasonore ;
définir, sur l'image ultrasonore, une région d'intérêt incluant la cible de détection de ligne B détectée ; et
**caractérisé en ce que** le procédé de contrôle comprend en outre
afficher, sur un moniteur, une ligne de suggestion de région d'intérêt constituée uniquement d'une partie d'une ligne de contour de la région d'intérêt, la partie étant située dans une partie plus superficielle que la ligne B.
